# EUROPEAN PATENT SPECIFICATION

(11) **EP 3 532 127 B1**
(45) Date of publication and mention of the grant of the patent: **08.04.2026**
(21) Application number: 17794614.2
(22) Date of filing: 25.10.2017
(51) Int. Cl.: A61M 5/142

(54) **ON-BODY INJECTOR**
INJEKTOR MIT KÖRPERKONTAKT
INJECTEUR DE CONTACT DE CORPS

(30) Priority: 25.10.2016 US 201662412365 P
(43) Date of publication of application: 04.09.2019
(73) Proprietor: Amgen Inc., Thousand Oaks, California 91320-1799 (US)
(72) Inventor: BOYAVAL, Margaux, Frances, Newbury Park, CA 91320 (US); CAMERON, Allan, Lee, Natick, MA 01760 (US); FLENDER, Gregg, Allen, Bedford, MA 01730 (US); BISCHOFF, Maxwell, Franklin, Somerville, MA 02144 (US)
(74) Representative: J A Kemp LLP
(86) International application number: PCT/US2017/058238
(87) International publication number: WO 2018/081234

(56) References cited:
- EP-A1- 3 020 328
- WO-A1-2010/029054
- US-A- 4 270 544
- US-A1- 2004 116 866
- US-A1- 2008 097 334
- US-A1- 2014 128 815
- US-A1- 2014 378 799
- US-A1- 2015 320 990

## Description

### FIELD OF THE DISCLOSURE

The present disclosure generally relates to drug delivery devices and, more particularly, a drug delivery device capable of being worn by a patient while the drug delivery device delivers a drug to the patient.

### BACKGROUND

Parenteral delivery of various drugs, i.e., delivery by means other than through the digestive track, has become a desired method of drug delivery for a number of reasons. This form of drug delivery by injection may enhance the effect of the substance being delivered and ensure that the unaltered medicine reaches its intended site at a significant concentration. Similarly, it is also possible with parenteral delivery to avoid undesirable side effects such as systemic toxicity associated with other routes of delivery. By bypassing the digestive system of a mammalian patient, one can avoid degradation of the active ingredients caused by the catalytic enzymes in the digestive tract and liver and ensure that a necessary amount of drug, at a desired concentration, reaches the targeted site.

Traditionally, manual syringes and injection pens have been employed for delivering parenteral drugs to a patient. More recently, parenteral delivery of liquid medicines into the body has been accomplished by administering bolus injections using a needle and reservoir, continuously by gravity driven dispensers, or via transdermal patch technologies. Bolus injections can require large individual doses. Continuous delivery of medicine through gravity-feed systems can compromise the patient's mobility and lifestyle, and limit therapy to simplistic flow rates and profiles. Transdermal patches often require specific molecular drug structures for efficacy, and the control of the drug administration through a transdermal patch can be severely limited.

Ambulatory infusion pumps have been developed for delivering liquid medicaments to a patient. These infusion devices have the ability to offer sophisticated fluid delivery profiles accomplishing bolus requirements, continuous infusion and variable flow rate delivery. These infusion capabilities usually result in better efficacy of the drug and therapy and less toxicity to the patient's system. Currently available ambulatory infusion devices are expensive, difficult to program and prepare for infusion, and tend to be bulky, heavy and fragile. Filling these devices can be difficult and require the patient to carry both the intended medication as well as filling accessories. The devices often require specialized care, maintenance, and cleaning to assure proper functionality and safety for their intended long-term use, and are not cost-effective for patients or healthcare providers.

As compared to syringes and injection pens, pump type delivery devices can be significantly more convenient to a patient, in that doses of the drug may be calculated and delivered automatically to a patient at any time during the day or night. Furthermore, when used in conjunction with metabolic sensors or monitors, pumps may be automatically controlled to provide appropriate doses of a fluidic medium at appropriate times of need, based on sensed or monitored metabolic levels. As a result, pump type delivery devices have become an important aspect of modern medical treatments of various types of medical conditions, such as diabetes, and the like.

While pump type delivery systems have been utilized to solve a number of patient needs, manually operated syringes and injection pens often remain a preferred choice for drug delivery as they now provide integrated safety features and can easily be read to identify the status of drug delivery and the end of dose dispensing. However, manually operated syringes and injection pens are neither universally applicable nor universally preferred for delivery of all drugs.

In recent years, wearable drug delivery devices, which are sometimes referred to as on-body injectors, have grown in applicability and preference. Like syringes, these wearable devices deliver drug by inserting a needle or cannula into the patient. But, unlike conventional syringes and pens, the patient or caregiver is not required to interact with the device after it is placed onto the patient's skin and activated. For some patients this removes the fear associated with manually inserting a needle or depressing a syringe plunger. Proper placement of the device throughout delivery, however, is important for proper and timely delivery of the drug and in some cases for avoiding patient discomfort. To facilitate proper placement, such delivery devices can be equipped with an adhesive layer that temporarily adheres to the patient's skin during delivery. This adhesive layer may initially be covered with a backing material that must be carefully removed prior to placing the device. And after delivery, the device with the adhesive layer must be carefully removed from the patient's skin, preferably in a comfortable manner without damaging the device itself.

WO 2010/029054 discloses a self-administration device for administering medication. US 2004/0116866 discloses an apparatus for attaching a patient infusion device. US 4 270 544 A discloses a further prior art device.

### BRIEF DESCRIPTION OF THE DRAWINGS

Fig. 1 is a first perspective view of a first embodiment of an on-body injector with adhesive applicator according to the principles of the present disclosure.
Fig. 2 is a second perspective view of the on-body injector of Fig. 1.
Fig. 3 is a first perspective view of a second embodiment of an on-body injector with adhesive applicator according to the principles of the present disclosure.
Fig. 4 is a second perspective view of the on-body injector of Fig. 3.
Fig. 5 is a first perspective view of a third embodiment of an on-body injector with adhesive applicator having a low tack pull tab according to the principles of the present disclosure.
Fig. 6 is a second perspective view of the on-body injector of Fig. 5.
Fig. 7 is a first perspective view of a fourth embodiment of an on-body injector with adhesive applicator and pull-ring according to the principles of the present disclosure.
Fig. 8 is a second perspective view of the on-body injector of Fig. 7 showing the pull-ring in a released position.
Fig. 9 is a first perspective view of a fifth embodiment of an on-body injector with adhesive applicator according to the principles of the present disclosure.
Fig. 10 is a second perspective view of the on-body injector of Fig. 9.
Fig. 11 is a first perspective view of a sixth embodiment of an on-body injector with adhesive applicator and pull-chord according to the principles of the present disclosure.
Fig. 12 is a second perspective view of the on-body injector of Fig. 11, showing the pull-chord in a released position and the injector separated from the adhesive applicator.
Fig. 13 is a first perspective view of a seventh embodiment of an on-body injector with adhesive applicator according to the principles of the present disclosure.
Fig. 14 is a second perspective view of the on-body injector of Fig. 13.
Fig. 15 is a first perspective view of a eighth embodiment of an on-body injector with adhesive applicator and pull-chord according to the principles of the present disclosure.
Fig. 16 is a second perspective view of the on-body injector of Fig. 15, showing the pull-chord in a released position and the injector separated from the adhesive applicator.
Fig. 17 is a first perspective view of a ninth embodiment of an on-body injector with adhesive applicator having a tear strip according to the principles of the present disclosure.
Fig. 18 is a second perspective view of the on-body injector of Fig. 17, showing the tear strip and injector in a released position.
Fig. 19 is a perspective view of a variation on the on-body injector of Figs. 17 and 18, showing an alternative arrangement for the tear strip.
Fig. 20 is a first perspective view of a tenth embodiment of an on-body injector with adhesive applicator and edge tabs according to the principles of the present disclosure.
Fig. 21 is a second perspective view of the on-body injector of Fig. 20, showing the edge tabs in a released position and the injector separated from the adhesive applicator.
Fig. 22 is a first perspective view of a eleventh embodiment of an on-body injector with adhesive applicator with pull-tabs according to the principles of the present disclosure.
Fig. 23 is a second perspective view of the on-body injector of Fig. 22.
Fig. 24 is a first perspective view of a twelfth embodiment of an on-body injector with adhesive applicator and grip tabs according to the principles of the present disclosure.
Fig. 25 is a second perspective view of the on-body injector of Fig. 24.

### DETAILED DESCRIPTION

Wearable injectors, which are often referred to as on-body injectors, can be applied to a patient's skin with an adhesive applicator. Selecting the configuration of the applicator requires consideration of at least the following two events: (1) applying the injector to the patient's skin and (2) removing the injector from the patient's skin. Each event includes different circumstances. For example, application may require a user to remove an adhesive backing layer to expose the adhesive layer, and then subsequently place the device against the skin with sufficient force to ensure a strong bond. Additionally, during application, it is important that the adhesive layer does not fold onto itself thereby rendering a portion of the adhesive layer unusable. This can be critical in situations where the injector includes a pre-filled injector because if the adhesive layer becomes unusable, the entire injector including the drug product in the injector may have to be discarded. Alternatively, the removal process requires some level of strength and dexterity to break the adhesive bond with the patient's skin. Moreover, some patients can be apprehensive in removing the device for fear that removing the adhesive and the inserted needle or cannula may cause discomfort. As such, disclosed herein are various novel arrangements for facilitating adherence of an on-body injector onto a patient's skin and subsequently removing the injector after drug delivery has occurred.

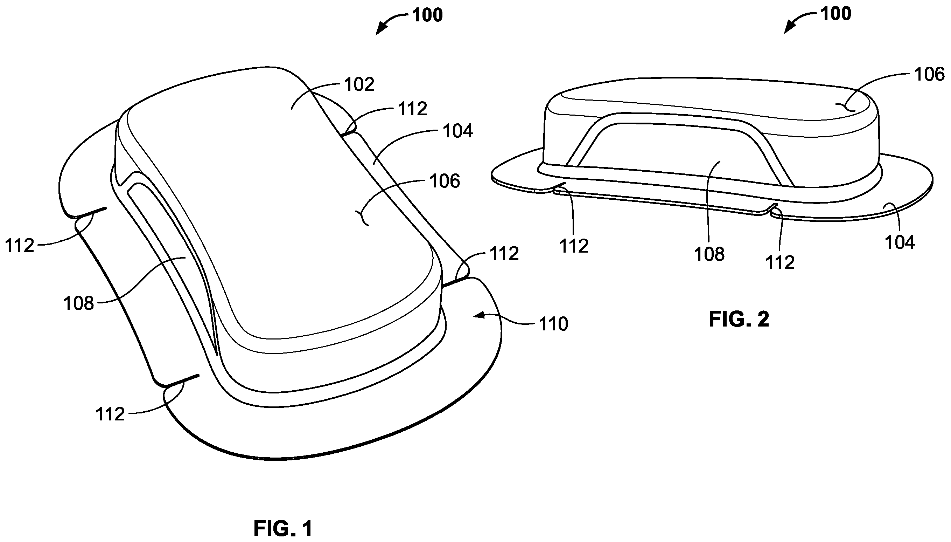

Figs. 1 and 2 depict a first embodiment of an on-body drug delivery device 100 including an injector 102 and adhesive applicator 104. The injector 102 can include a pre-filled, pre-loaded drug delivery device having an external housing 106 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 102 of this embodiment can include side grips 108 molded into side walls of the housing 106 for assisting a user with grasping the device 100 for placement and/or removal. In the depicted version, the side grips 108 include elongated recesses molded into the housing 106 at a location between the bottom and the top surfaces of the housing 106. The adhesive applicator 104 of this embodiment includes a non-woven material fixed to a bottom surface of the injector 102 and includes a dimension that is larger than the injector 102 itself. So configured, the adhesive applicator 104 includes a perimeter portion 110 that encircles the injector 102. The perimeter portion 110 also includes a plurality of radial slits 112 that allow the perimeter portion to more easily deform to meet the contours of the patient's application site. Finally, a bottom surface 114 of the adhesive applicator 104 includes a layer of an adhesive (not shown) and an adhesive backing material (not shown). So configured, to apply the device 100 to a patient, a user must first remove the adhesive backing layer to expose the adhesive layer. Then, the entire device can be placed against a patient's injector site such that exposed adhesive on the adhesive applicator 104 adheres to the patient's skin. After injection, a user can simply peel the perimeter portion 110 of the adhesive applicator 104 away from the skin with one hand, while grasping the injector 102 at the side grips 108 with the other hand and pulling away from the patient.

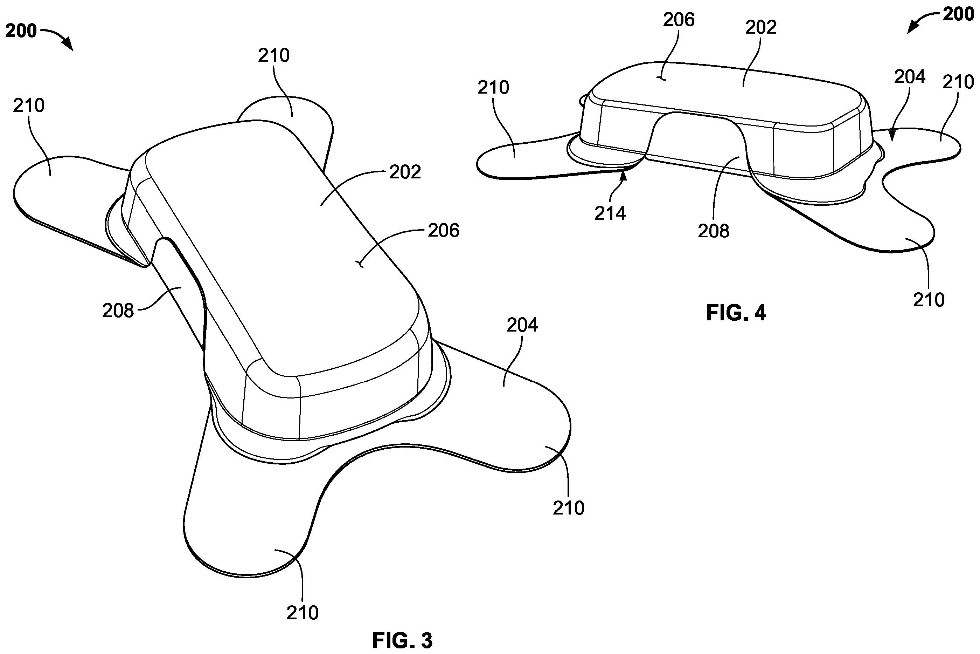

Figs. 3 and 4 depict a second embodiment of an on-body drug delivery device 200 including an injector 202 and adhesive applicator 204. The injector 202 can include a pre-filled, pre-loaded drug delivery device having an external housing 206 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 202 of this embodiment can include side grips 208 molded into side walls of the housing 206 for assisting a user with grasping the device 200 for placement and/or removal. In the depicted version, the side grips 208 include elongated recesses molded into the housing 106 at a location adjacent the bottom surface of the housing 206. The adhesive applicator 204 of this embodiment includes a non-woven material fixed to a bottom surface of the injector 202 and includes a plurality of tabs 210 that extend beyond a perimeter of the injector 102 itself. In the depicted version, the adhesive applicator 204 includes an X-shape so that each tab 210 extends at an angle from a respective corner of the injector 202. This specific configuration can assist the applicator 204 to deform and meet the contours of the patient's application site. Finally, a bottom surface 214 of the adhesive applicator 204 includes a layer of an adhesive (not shown) and an adhesive backing material (not shown). So configured, to apply the device 200 to a patient, a user must first remove the adhesive backing layer to expose the adhesive layer. Then, the entire device can be placed against a patient's injector site such that exposed adhesive on the adhesive applicator 204 adheres to the patient's skin. After injection, a user can simply peel the tabs 210 of the adhesive applicator 204 away from the skin with one hand, while grasping the injector 202 at the side grips 208 with the other hand and pulling away from the patient. As mentioned above, the side grips 208 of this embodiment are located adjacent to the bottom surface of the injector 202 and, in some version, can actually extend around the bottom surface of the injector 202. So configured, this arrangement can enable a user to more easily insert his/her fingers between the injector 202 and the patient's skin to assist with removal.

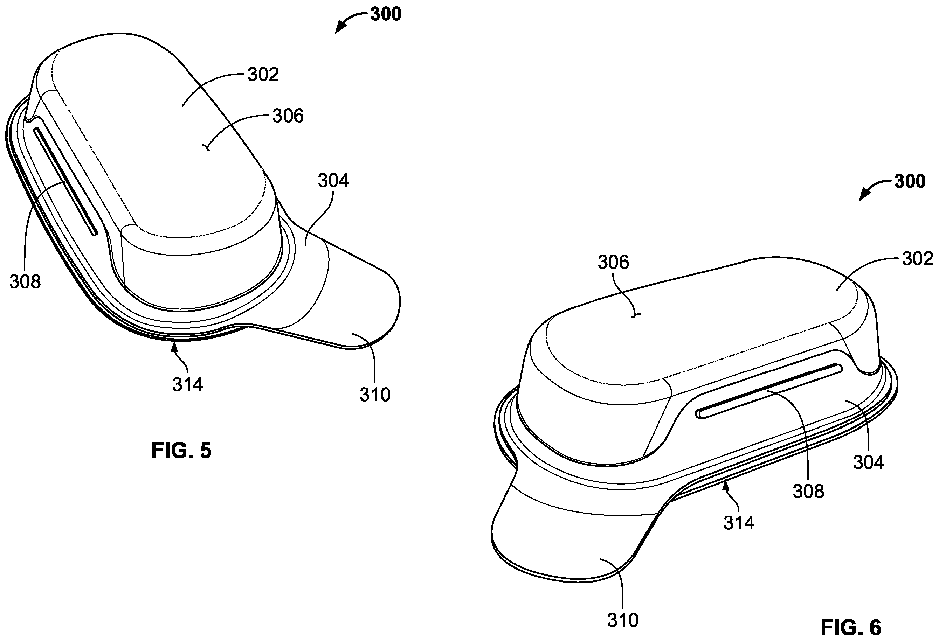

Figs. 5 and 6 depict a third embodiment of an on-body drug delivery device 300 including an injector 302 and adhesive applicator 304. The injector 302 can include a pre-filled, pre-loaded drug delivery device having an external housing 306 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 302 of this embodiment can include side grips 308 molded into a paper, plastic, silicone, rubber, or other suitable material forming a label or skin that can encase the housing 306 for assisting a user with grasping the device 300 for placement and/or removal. The label or skin can have an adhesive on itself for adhering to the housing 306, or it can sit upon another substrate (such as a non-woven substrate) that has an adhesive applied thereto. In the depicted version, the side grips 308 include elongated ribs disposed at a location between the bottom and top surfaces of the housing 306. The adhesive applicator 304 of this embodiment can include a non-woven material (or other suitable material) fixed to a bottom surface of the injector 302 and includes a single pull tab 310 extending beyond a perimeter of the injector 302 itself. In the depicted version, the pull tab 310 extends at an angle from a corner of the injector 302. Finally, a bottom surface 314 of the adhesive applicator 304 including the pull tab 310 includes a layer of an adhesive (not shown) and an adhesive backing material (not shown). In some versions, the adhesive applicator 304 may include the same adhesive on the pull tab 310 as the remainder of the applicator 304, and in other versions, one adhesive on the underside of the pull tab 310 may have less tack (e.g., adhesive strength) than another adhesive on the underside of the remainder of the applicator 304.

To apply the device 300 to a patient, a user must first remove the adhesive backing layer to expose the adhesive layer. Then, the entire device can be placed against a patient's injector site such that exposed adhesive on the adhesive applicator 304 adheres to the patient's skin. After injection, a user can simply peel the pull tab 310 of the adhesive applicator 304 away from the skin with one hand, while grasping the injector 302 at the side grips 308 with the other hand and pulling away from the patient. As mentioned above, the pull tab 310 in some versions may have less tack than the remainder of the adhesive applicator 304, and this configuration may assist a user in initiating removal of the device 300 from the patient's skin. Furthermore, as shown in Figs. 5 and 6, the pull tab 310 in some embodiments may be of a distinct color or shading such as to draw the user's attention to the pull tab 310 during removal. As such, this coloring or shading such serve as an indicator to communicate with the user.

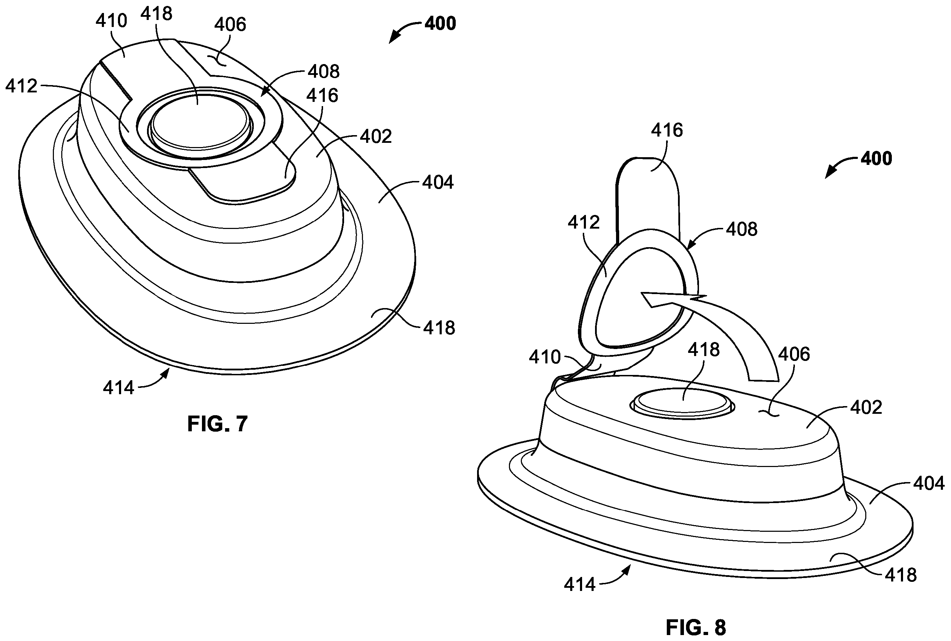

Figs. 7 and 8 depict a fourth embodiment of an on-body drug delivery device 400 including an injector 402 and adhesive applicator 404. The injector 402 can include a pre-filled, pre-loaded drug delivery device having an external housing 406 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 402 of this embodiment includes a pull ring 408 for assisting a user with removing the device 400 from the patient after drug delivery. In the depicted version, the pull ring 408 includes elongated arm 410, a ring 412 and a peel tab 416. One end of the arm 410 is secured to the injector 402 or adhesive applicator 404, and the ring 412 is secured between the arm 410 and peel tab 416. Thus, as shown in Fig. 7, when the device 400 occupies a pre-drug-delivery state, the pull ring 408 lays on the top surface of the injector body 406, being retained in this position by an adhesive located on the peel tab 416. In the depicted version, the injector 402 includes an activation button 418 on the top surface, so the ring 412 of the ring pull 408 accommodates that button 418.

The adhesive applicator 404 of this embodiment includes a non-woven material fixed to a bottom surface of the injector 402 and includes a perimeter portion 418 that extends beyond a perimeter of the injector 402 itself. Finally, a bottom surface 414 of the adhesive applicator 404 includes a layer of an adhesive (not shown) and an adhesive backing material (not shown). So configured, to apply the device 400 to a patient, a user must first remove the adhesive backing layer to expose the adhesive layer. Then, the entire device can be placed against a patient's injector site such that exposed adhesive on the adhesive applicator 404 adheres to the patient's skin. After injection, a user peels the peel tab 416 of the pull ring 408 away from the top surface of the injector 402 thereby releasing the pull ring 408 into the position shown in Fig. 8. In this position, the user can insert a finger through the ring 412 and pull the entire device 400 off of the patient. In some versions, as the user pulls the pull ring 408 with one hand, he/she can slightly peel the perimeter portion 418 of the adhesive applicator 404 away from the skin to facilitate removal as well. One benefit of the pull ring configuration disclosed in this embodiment is that is that it can provide those users with limited strength and dexterity additional leverage and force for removal.

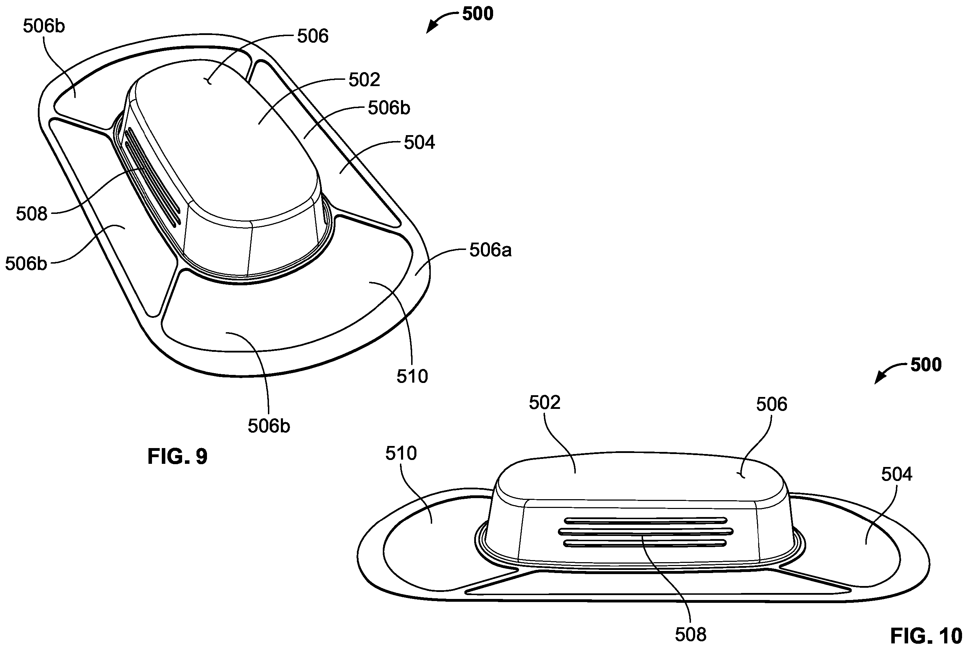

Figs. 9 and 10 depict a fifth embodiment of an on-body drug delivery device 500 including an injector 502 and adhesive applicator 504. The injector 502 can include a pre-filled, pre-loaded drug delivery device having an external housing 506 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 502 of this embodiment can include side grips 508 molded into side walls of the housing 506 for assisting a user with grasping the device 500 for placement and/or removal. In the depicted version, the side grips 508 include a plurality of elongated and parallel ribs and recesses formed on the housing 506 at a location between the bottom and top surfaces of the housing 506. The adhesive applicator 504 of this embodiment includes a non-woven material fixed to a bottom surface of the injector 502 and includes a perimeter portion 510 that extends beyond a perimeter of the injector 502 itself. In the depicted version, the adhesive applicator 504 includes a skeletal structure 506a interconnecting a plurality of primary bonding structures 506b. The skeletal structure 506a may include a more rigid structural material than the primary bonding structures 506b, which can include a very flexible bonding material for good attachment to a patient's skin. The stiffer skeletal structure 506a can provide some added benefit by allowing a user to more easily remove the backing material. So configured, to apply the device 500 to a patient, a user must first remove the adhesive backing layer to expose the adhesive layer. Then, the entire device can be placed against a patient's injector site such that exposed adhesive on the adhesive applicator 504 adheres to the patient's skin. After injection, a user can simply peel the perimeter portion 510 of the adhesive applicator 504 away from the skin with one hand, while grasping the injector 502 at the side grips 508 with the other hand and pulling away from the patient.

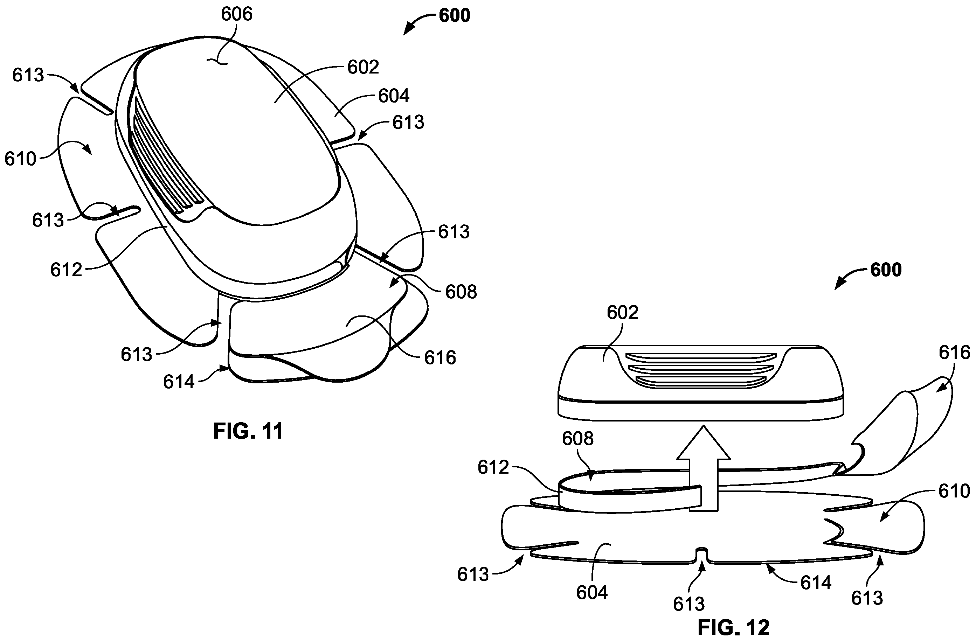

Figs. 11 and 12 depict a sixth embodiment of an on-body drug delivery device 600 including an injector 602 and adhesive applicator 604. The injector 602 can include a pre-filled, pre-loaded drug delivery device having an external housing 606 that can contain, for example, a drug reservoir filled (or adapted to be filled) with a drug, a needle and/or a cannula for insertion into a patient, and a drive mechanism for urging the drug out of the reservoir and into the patient as is generally known in the art. The adhesive applicator 604 of this embodiment includes a non-woven material fixed to a bottom surface of the injector 602 and includes a perimeter portion 610 that extends beyond a perimeter of the injector 602 itself. The perimeter portion 610 also includes a plurality of radial slits 613 that allow the perimeter portion 610 to more easily deform to meet the contours of the patient's application site. A bottom surface 614 of the adhesive applicator 604 includes a layer of an adhesive (not shown) and an adhesive backing material (not shown).

As shown, the injector 602 further includes a pull chord 408 for assisting a user with removing the device 600 from the patient after drug delivery. In the depicted version, the pull chord 608 includes a ring 612 and a peel tab 616. As shown in Fig. 11, when the device 600 occupies a pre-drug-delivery state, the pull chord 608 secures the injector 606 to the adhesive applicator 604. More specifically, the ring 612 is attached between the injector 602 and the perimeter portion 610 of the adhesive applicator 604. As such, the adhesive applicator 604 can safely properly secure the injector 602 in position during drug delivery.

After injection, however, a user grasps the peel tab 616 of the pull chord 408 and pulls it away from the injector 602, as illustrated in Fig. 12. As the user pulls the tab 616, this causes the ring 612 to detach from the perimeter of the injector 602 and the adhesive applicator 604 in a manner similar to removing a plastic cap from a sealed milk container. The ring 612 can be attached to the injector 602 and applicator 604 with a perforated seam or some other similar mechanism. With the pull chord 608 removed, a user can grasp the injector 602 and easily pull it away from the patient's skin. In the depicted version, the adhesive applicator 604 can be a continuous sheet of material attached to the patient's skin while the bottom surface of the injector 602 includes a second removable adhesive layer bonding the injector 602 to the adhesive applicator 604. In other versions, the adhesive applicator 604 may have a hole in the center of it and the bottom surface of the injector 602 can bond directly to the patient's skin. In such a version, the adhesive applicator beneath the injector 602 may include an adhesive having less tack (e.g., adhesive strength) than the remaining portions of the adhesive applicator 604. Subsequently, the user may peel the remaining portions of the adhesive applicator 404 away from the patient's skin. This two-step removal process may provide the user with a level of comfort knowing that the injector 402 can be easily removed from the skin in a direction that is substantially orthogonal to the injection site, thereby alleviating any fears that may exist regarding discomfort that can be caused from removing the needle or cannula at an angle less than ninety-degrees. Moreover, this process may also enable the user to utilized a liquid solution such as soapy water, for example, to release the stronger adhesive carried by the adhesive applicator 604.

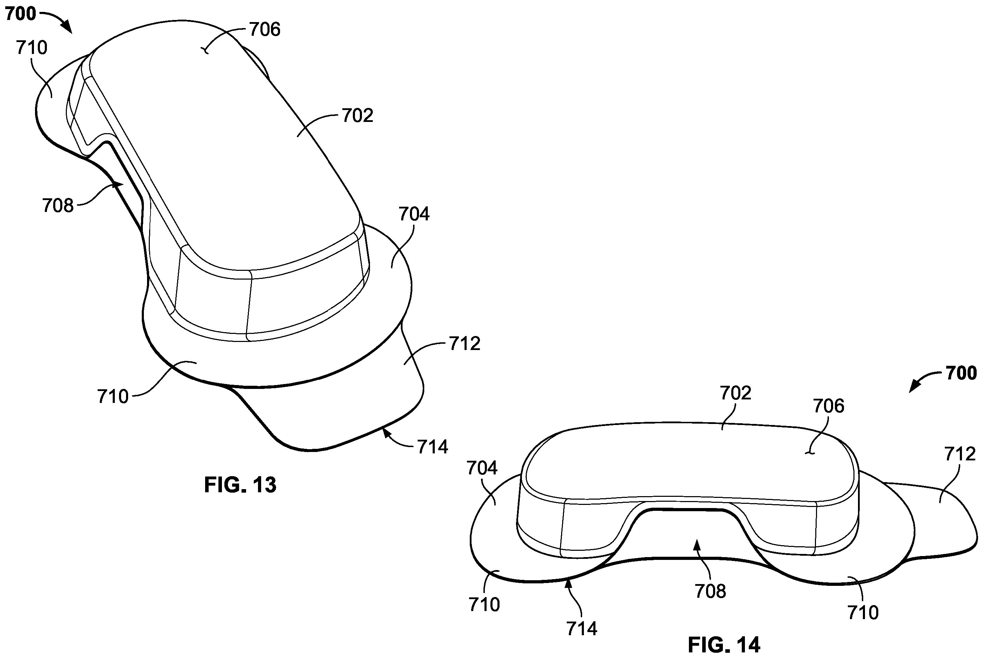

Figs. 13 and 14 depict a seventh embodiment of an on-body drug delivery device 700 combining aspects of the second and third embodiments described above. The device 700 includes an injector 702 and adhesive applicator 704. The injector 702 can include a pre-filled, pre-loaded drug delivery device having an external housing 706 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 702 of this embodiment can include side grips 708 molded into side walls of the housing 706 for assisting a user with grasping the device 700 for placement and/or removal. In the depicted version, the side grips 708 include elongated recesses molded into the housing 706 at a location adjacent the bottom surface of the housing 706. In some versions, the side grips 708 can be formed of a soft or resilient material to aid in the gripping function both during application and removal of the injector 702. The adhesive applicator 704 of this embodiment includes a non-woven material fixed to a bottom surface of the injector 702 and includes a plurality of tabs 710 that extend beyond a perimeter of the injector 702 itself. In the depicted version, the adhesive applicator 704 includes two tabs, each extending from opposite ends of the deice 700. One of the tabs 710 further includes a peel flange 712 extending further away from the injector 700 for facilitating removal. Finally, a bottom surface 714 of the adhesive applicator 704 includes a layer of an adhesive (not shown) and an adhesive backing material (not shown). In some versions, the adhesive applicator 704 may include the same adhesive on the peel flange 712 as the remainder of the applicator 704, and in other versions, one adhesive on the underside of the pull flange 712 may have less tack (e.g., adhesive strength) than another adhesive on the underside of the remainder of the applicator 704.

To apply the device 700 to a patient, a user must first remove the adhesive backing layer to expose the adhesive layer. Then, the entire device can be placed against a patient's injector site such that exposed adhesive on the adhesive applicator 704 adheres to the patient's skin. After injection, a user can simply peel the peel flange 712 of the adhesive applicator 704 away from the skin with one hand, while grasping the injector 702 at the side grips 708 with the other hand and pulling away from the patient. As mentioned above, the side grips 708 of this embodiment are located adjacent to the bottom surface of the injector 702 and, in some version, can actually extend around the bottom surface of the injector 702. So configured, this arrangement can enable a user to more easily insert his/her fingers between the injector 702 and the patient's skin to assist with removal. Also as mentioned above, the peel flange 712 in some versions may have less tack than the remainder of the adhesive applicator 704, and this configuration may assist a user in initiating removal of the device 700 from the patient's skin. Furthermore, as shown in Figs. 13 and 14, the pull flange 712 in some embodiments may be of a distinct color or shading such as to draw the user's attention to the pull flange 712 during removal. As such, this coloring or shading such serve as an indicator to communicate with the user.

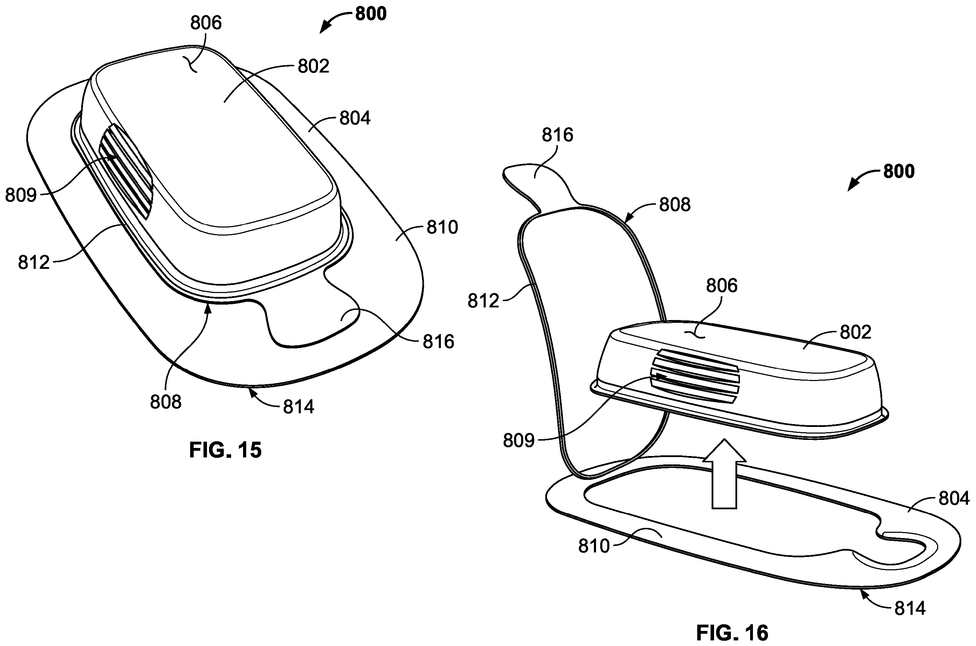

Figs. 15 and 16 depict a eighth embodiment of an on-body drug delivery device 800 including an injector 802 and adhesive applicator 804. The injector 802 can include a pre-filled, pre-loaded drug delivery device having an external housing 806 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 802 of this embodiment can include side grips 809 molded into side walls of the housing 806 for assisting a user with grasping the device 800 for placement and/or removal. In the depicted version, the side grips 809 include elongated recesses and/or ribs molded into the housing 806 at a location adjacent the bottom surface of the housing 806. The adhesive applicator 804 of this embodiment includes a non-woven material fixed to a bottom surface of the injector 802 and includes a perimeter portion 810 that extends beyond a perimeter of the injector 802 itself. A bottom surface 814 of the adhesive applicator 804 includes a layer of an adhesive (not shown) and an adhesive backing material (not shown).

As shown, the injector 802 further includes a pull chord 808 for assisting a user with removing the device 800 from the patient after drug delivery. In the depicted version, the pull chord 608 includes a ring 812 and a peel tab 816. As shown in Fig. 15, when the device 800 occupies a pre-drug-delivery state, the pull chord 808 secures the injector 806 to the adhesive applicator 804. More specifically, the ring 812 is attached between the injector 802 and the perimeter portion 810 of the adhesive applicator 804. As such, the adhesive applicator 804 can safely properly secure the injector 802 in position during drug delivery.

After injection, however, a user grasps the peel tab 816 of the pull chord 808 and pulls it away from the injector 802, as illustrated in Fig. 16. As the user pulls the tab 916, this causes the ring 912 to detach from the perimeter of the injector 902 and the adhesive applicator 904 in one straight continuous motion. The ring 812 can be attached to the injector 802 and applicator 804 with a perforated seam or some other similar mechanism. With the pull chord 808 removed, a user can grasp the injector 802 and easily pull it away from the patient's skin. As shown in Fig. 16, the center of the adhesive applicator 804 in this version includes a hole for the injector 802 to attach directly to the patient's skin. In some versions, the adhesive applicator beneath the injector 802 may include an adhesive having less tack (e.g., adhesive strength) than the remaining portions of the adhesive applicator 804. In some versions, the tack may be the same. In yet other versions, the adhesive applicator 804 extends continuously beneath the injector 802 and the device 800 includes a second removable adhesive layer bonding the injector 802 to the adhesive applicator 804, as shown for example in the embodiment described above in Figs. 11 and 12. Subsequently, the user may peel the remaining portions of the adhesive applicator 804 away from the patient's skin. This two-step removal process may provide the user with a level of comfort knowing that the injector 802 can be easily removed from the skin in a direction that is substantially orthogonal to the injection site, thereby alleviating any fears that may exist regarding discomfort that can be caused from removing the needle or cannula at an angle less than ninety-degrees. Moreover, this process may also enable the user to utilized a liquid solution such as soapy water, for example, to release the stronger adhesive carried by the adhesive applicator 804.

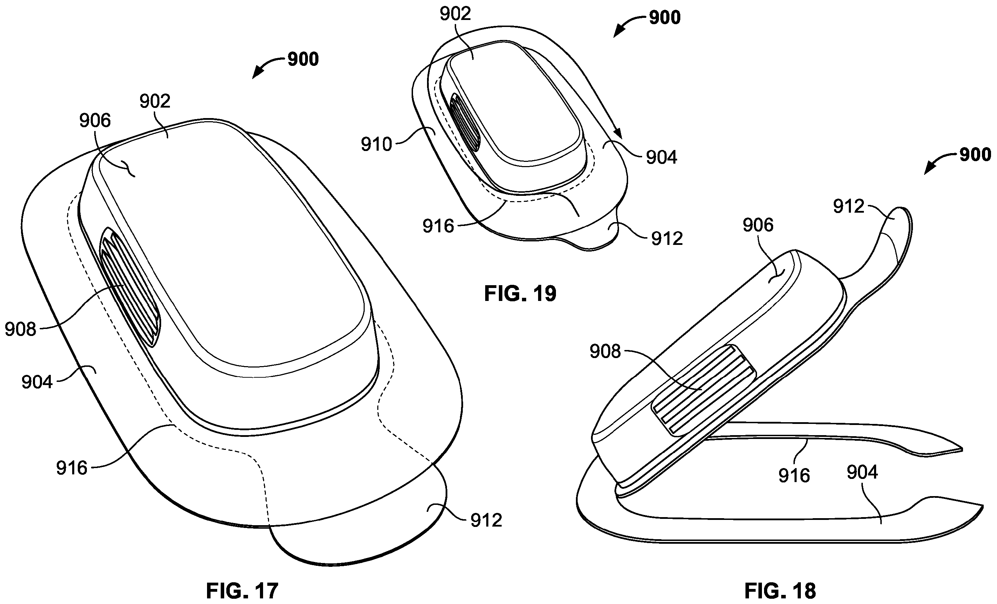

Figs. 17 and 18 depict a ninth embodiment of an on-body drug delivery device 900 including an injector 902 and adhesive applicator 904. The injector 902 can include a pre-filled, pre-loaded drug delivery device having an external housing 906 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 902 of this embodiment can include side grips 908 molded into side walls of the housing 906 for assisting a user with grasping the device 900 for placement and/or removal. In the depicted version, the side grips 908 include elongated recesses molded into the housing 906 at a location adjacent the bottom surface of the housing 906. The adhesive applicator 904 of this embodiment includes a non-woven material fixed to a bottom surface of the injector 902 and includes a perimeter portion 910 that extends beyond a perimeter of the injector 902 itself.

In the depicted version, the adhesive applicator 904 further includes a single pull tab 912 and perforated seam 916 extending around the injector 902. Finally, a bottom surface 914 of the adhesive applicator 904 includes a layer of an adhesive (not shown) and an adhesive backing material (not shown). In some versions, the adhesive applicator 904 may include the same adhesive on the pull tab 912 as the remainder of the applicator 904, and in other versions, one adhesive on the underside of the pull tab 912 may have less tack (e.g., adhesive strength) than another adhesive on the underside of the remainder of the applicator 904.

To apply the device 900 to a patient, a user must first remove the adhesive backing layer to expose the adhesive layer. Then, the entire device can be placed against a patient's injector site such that exposed adhesive on the adhesive applicator 904 adheres to the patient's skin. After injection, a user can simply peel the pull tab 912 of the adhesive applicator 904 away from the skin with one hand. As the poll tab 912 is pulled, the adhesive applicator 904 tears along the perforated seam 916 allowing the pull force to also remove the injector 902 from the patient's skin as shown in Fig. 18. Continued pulling force remove the entire perimeter portion 910 from the patient in one clean movement. Also as mentioned above, the pull tab 912 in some versions may have less tack than the remainder of the adhesive applicator 904, and this configuration may assist a user in initiating removal of the device 900 from the patient's skin. Furthermore, as shown in Figs. 17 and 18, the pull tab 912 in some embodiments may be of a distinct color or shading such as to draw the user's attention to the pull tab 912 during removal. As such, this coloring or shading such serve as an indicator to communicate with the user.

Fig. 19 depicts a variation on the embodiment just described in reference to Figs. 17 and 18. Like the device 900 in Figs. 17 and 18, the device 900 in Fig. 19 includes an injector 902 and adhesive application 904, which includes a pull tab 912 and a perforated seam 916 surrounding the injector 902. However, in contrast to that described in Figs. 17 and 18, pulling the pull tab 912 of the device in Fig. 19 caused the adhesive applicator 904 to tear along the perforated seam 916, around the injector 902, first removing the perimeter portion 910 of the applicator 904 from the patient. Meanwhile, the injector 902 remains adhered to the patient. Then, in a second subsequent act, the user can remove the injector 902 separately.

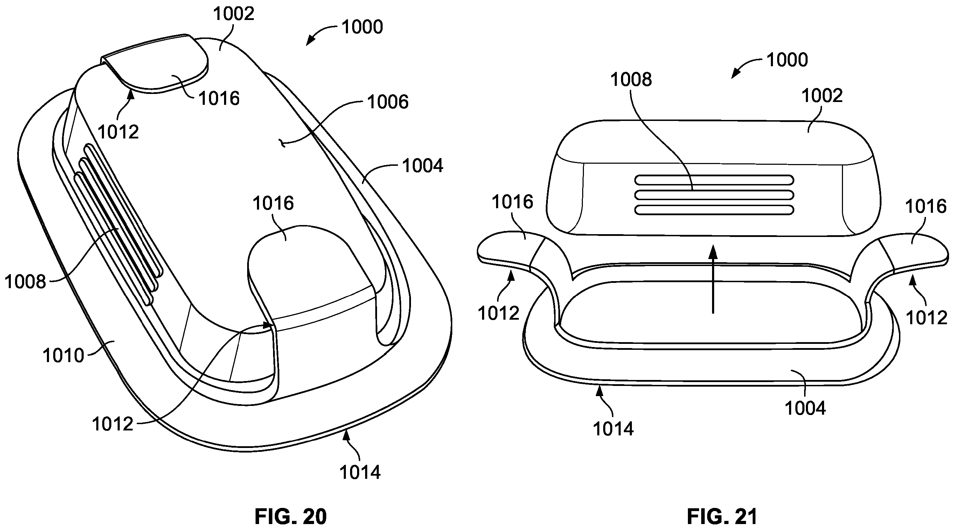

Figs. 20 and 21 depict a tenth embodiment of an on-body drug delivery device 1000 including an injector 1002 and adhesive applicator 1004. The injector 1002 can include a pre-filled, pre-loaded drug delivery device having an external housing 1006 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 1002 of this embodiment can include side grips 1008 molded into side walls of the housing 1006 for assisting a user with grasping the device 1000 for placement and/or removal. In the depicted version, the side grips 1008 include elongated ribs and/or recesses molded into the housing 1006 at a location between the bottom and the top surfaces of the housing 1006. The adhesive applicator 1004 of this embodiment includes a non-woven material fixed to a bottom surface of the injector 1002 and includes a dimension that is larger than the injector 1002 itself. So configured, the adhesive applicator 1004 includes a perimeter portion 1010 that encircles the injector 1002. Finally, a bottom surface 1014 of the adhesive applicator 104 includes a layer of an adhesive (not shown) and an adhesive backing material (not shown). As also shown in Figs. 20 and 21, the device 1000 includes a pair of edge tabs 1012 extending up from opposite sides of the perimeter portion 1010 of the adhesive applicator 1004. Each edge tab 1012 terminates in a pull tab 1016. In a pre-drug-delivery state depicted in Fig. 20, the edge tabs 1012 are folded over and down onto the injector 1002 such that the pull tabs 1016 adhere to its top surface. In some versions, the portion of the edge tabs 1012 between the pull tabs 1016 and the perimeter portion 1010 of the adhesive applicator 1004 may also include adhesive and be bonded to the side surface of the injector 1002. So configured, the edge tabs 1012 securely fix the injector 1002 in position relative to the adhesive applicator 1004 before and during drug delivery.

To apply the device 1000 to a patient, a user must first remove the adhesive backing layer to expose the adhesive layer. Then, the entire device can be placed against a patient's injector site such that exposed adhesive on the adhesive applicator 1004 adheres to the patient's skin. After injection, a user grasps the pull tabs 1016 of the edge tabs 1012 and pull them away from the injector 1002, as shown in Fig. 21. With the edge tabs 1012 detached from the injector 1002, a user can grasp the injector 1002 and pull is straight away from the patient's skin. Subsequently, the user can peel the perimeter portion 1010 of the adhesive applicator 1004 off of the patient's skin without having to be concerned with the position of the needle or cannula associated with the injector 1002. In the version depicted in Fig. 21, the adhesive applicator 1004 has a hole in the center of it when the injector 1002 is removed. In this version, the bottom surface of the injector 1002 directly carries the remainder of the adhesive applicator 1004. In other versions, the adhesive applicator 1004 extends continuously beneath the injector 1002 and the injector 1002 carries a secondary adhesive that bond to the top surface of the adhesive applicator layer 1008. As shown in Figs. 20 and 21, the pull tabs 1016 of the edge tabs 1012 in some embodiments may be of a distinct color or shading such as to draw the user's attention to the pull tabs 1016 during removal. As such, this coloring or shading such serve as an indicator to communicate with the user.

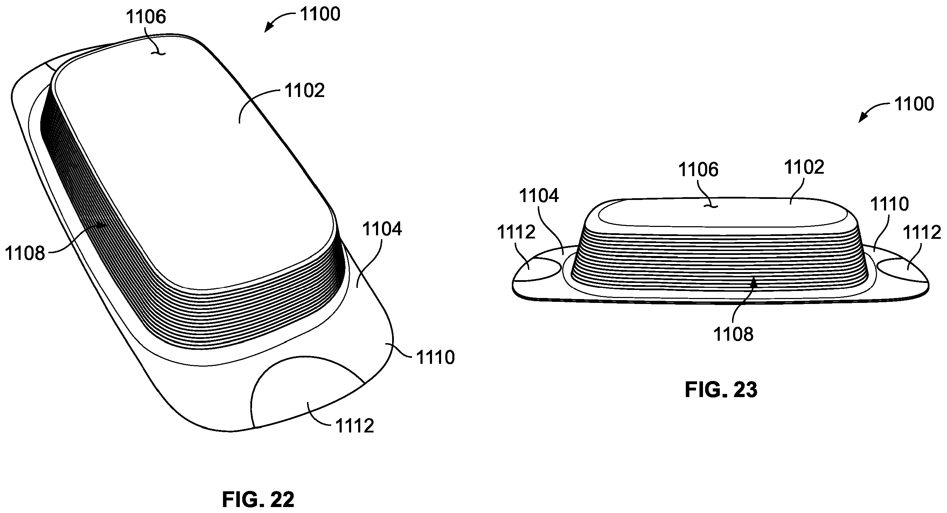

Figs. 22 and 23 depict an eleventh embodiment of an on-body drug delivery device 1100 including an injector 1102 and adhesive applicator 1104. The injector 1102 can include a pre-filled, pre-loaded drug delivery device having an external housing 1106 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 1102 of this embodiment can include side grips 1108 molded into side walls of the housing 1106 for assisting a user with grasping the device 1100 for placement and/or removal. In the depicted version, the side grips 1108 include elongated recesses or ribs molded into the housing 1106 at a location between the bottom and the top surfaces of the housing 1106, and encircling the entirety of the housing 1106. The adhesive applicator 1104 of this embodiment includes a non-woven material fixed to a bottom surface of the injector 1102 and includes a dimension that is larger than the injector 1102 itself. So configured, the adhesive applicator 1104 includes a perimeter portion 1110 that encircles the injector 1102. The perimeter portion 1110 also includes a pair of distinct pull tab portions 112. Finally, a bottom surface 1114 of the adhesive applicator 1104 includes a layer of an adhesive (not shown) and an adhesive backing material (not shown). So configured, to apply the device 1100 to a patient, a user must first remove the adhesive backing layer to expose the adhesive layer. Then, the entire device can be placed against a patient's injector site such that exposed adhesive on the adhesive applicator 1104 adheres to the patient's skin. After injection, a user can simply grasp the pull tab portions 1112 of the perimeter portion 1110 of the adhesive applicator 1104 and pull away from the skin. In some versions, the adhesive beneath the pull tab portions 1112 of the adhesive applicator 1004 can have less tack (e.g., adhesive strength) than the adhesive on the remainder of the adhesive applicator 1004. Also as shown in Figs. 22 and 23, the pull tabs 1112 in some embodiments may be of a distinct color or shading such as to draw the user's attention to the pull tabs 1112 during removal. As such, this coloring or shading serves as an indicator to communicate with the user.

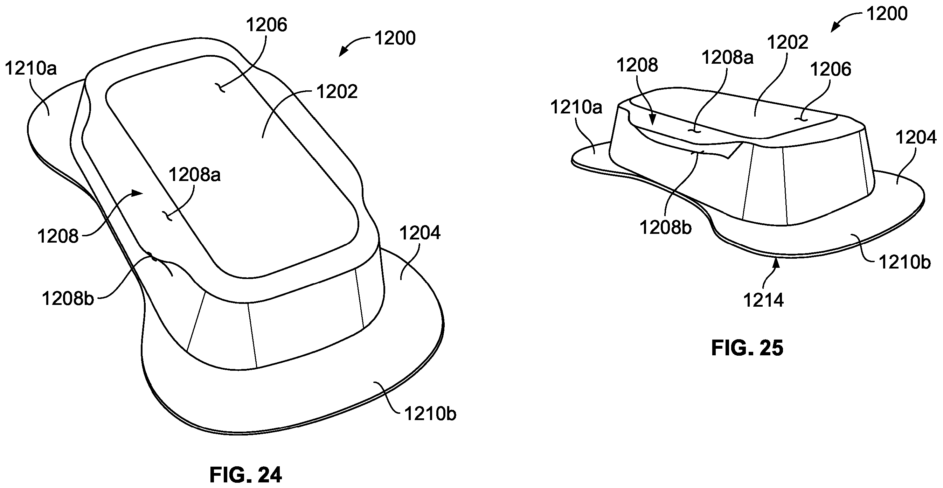

Figs. 24 and 25 depict an twelfth embodiment of an on-body drug delivery device 1200 including an injector 1202 and adhesive applicator 1204. The injector 1202 can include a pre-filled, pre-loaded drug delivery device having an external housing 1206 that can contain, for example, a drug reservoir filled (or adapted to be filled) with a drug, a needle and/or a cannula for insertion into a patient, and a drive mechanism for urging the drug out of the reservoir and into the patient as is generally known in the art. As shown, the injector 1202 of this embodiment can include side grips 1208 molded into side walls of the housing 1206 for assisting a user with grasping the device 1200 for placement and/or removal. In the depicted version, the side grips 1208 include elongated ribs or protrusions extending out from the housing 1206 at a location adjacent the top surfaces of the housing 1206. In the depicted version, the side grips 1208 include smoothed contoured upper surfaces 1208a and undercut lower surfaces 1208b to assist gripping. The adhesive applicator 1204 of this embodiment includes a non-woven material fixed to a bottom surface of the injector 1202 and includes a dimension that is larger than the injector 1202 itself. So configured, the adhesive applicator 1204 includes a perimeter portion 1210 that has two tab portions 1210a and 1210b extending from opposite ends of the injector 1202. Finally, a bottom surface 1214 of the adhesive applicator 1204 includes a layer of an adhesive (not shown) and an adhesive backing material (not shown). So configured, to apply the device 1200 to a patient, a user must first remove the adhesive backing layer to expose the adhesive layer. Then, the entire device can be placed against a patient's injector site such that exposed adhesive on the adhesive applicator 1204 adheres to the patient's skin. After injection, a user can peel either or both of the tab portions 1212 of the perimeter portion 1210 of the adhesive applicator 1204 away from the skin.

Many of the foregoing embodiments have overlapping features and aspects, and a person of ordinary skill reading this disclosure would understand that an embodiment of the present disclosure can also include unique combinations of those features and concepts not expressly disclosed herein. Specifically, a device within the scope of the present disclosure may benefit from a side gripping arrangement disclosed in any one or more of the other embodiments, including combinations of side gripping arrangements from various embodiments. Additionally, a device within the scope of the present disclosure may benefit from an adhesive applicator disclosed in any one or more of the other embodiments, including combinations of adhesive applicator arrangements from various embodiments. Furthermore, a device within the scope of the present disclosure may benefit from a tab or ring arrangement disclosed in any one or more of the other embodiments, including combinations of tab or ring arrangements from various embodiments.

The above description describes various systems and methods for use with a drug delivery device. It should be clear that the system, drug delivery device or methods can further comprise use of a medicament listed below with the caveat that the following list should neither be considered to be all inclusive nor limiting. The medicament will be contained in a reservoir. In some instances, the reservoir is a primary container that is either filled or pre-filled for treatment with the medicament. The primary container can be a cartridge or a pre-filled syringe.

For example, the drug delivery device or more specifically the reservoir of the device may be filled with colony stimulating factors, such as granulocyte colony-stimulating factor (G-CSF). Such G-CSF agents include, but are not limited to, Neupogen^{®} (filgrastim) and Neulasta^{®} (pegfilgrastim). In various other embodiments, the drug delivery device may be used with various pharmaceutical products, such as an erythropoiesis stimulating agent (ESA), which may be in a liquid or a lyophilized form. An ESA is any molecule that stimulates erythropoiesis, such as Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin zeta, epoetin theta, and epoetin delta, as well as the molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,986,047; 6,583,272; 7,084,245; and 7,271,689; and PCT Publication Nos. WO 91/05867; WO 95/05465; WO 96/40772; WO 00/24893; WO 01/81405; and WO 2007/136752.

An ESA can be an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, epoetin alfa, epoetin beta, epoetin delta, epoetin omega, epoetin iota, epoetin zeta, and analogs thereof, pegylated erythropoietin, carbamylated erythropoietin, mimetic peptides (including EMP1/hematide), and mimetic antibodies. Exemplary erythropoiesis stimulating proteins include erythropoietin, darbepoetin, erythropoietin agonist variants, and peptides or antibodies that bind and activate erythropoietin receptor (and include compounds reported in U.S. Publication Nos. 2003/0215444 and 2006/0040858) as well as erythropoietin molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,830,851; 5,856,298; 5,986,047; 6,030,086; 6,310,078; 6,391,633; 6,583,272; 6,586,398; 6,900,292; 6,750,369; 7,030,226; 7,084,245; and 7,217,689; U.S. Publication Nos. 2002/0155998; 2003/0077753; 2003/0082749; 2003/0143202; 2004/0009902; 2004/0071694; 2004/0091961; 2004/0143857; 2004/0157293; 2004/0175379; 2004/0175824; 2004/0229318; 2004/0248815; 2004/0266690; 2005/0019914; 2005/0026834; 2005/0096461; 2005/0107297; 2005/0107591; 2005/0124045; 2005/0124564; 2005/0137329; 2005/0142642; 2005/0143292; 2005/0153879; 2005/0158822; 2005/0158832; 2005/0170457; 2005/0181359; 2005/0181482; 2005/0192211; 2005/0202538; 2005/0227289; 2005/0244409; 2006/0088906; and 2006/0111279; and PCT Publication Nos. WO 91/05867; WO 95/05465; WO 99/66054; WO 00/24893; WO 01/81405; WO 00/61637; WO 01/36489; WO 02/014356; WO 02/19963; WO 02/20034; WO 02/49673; WO 02/085940; WO 03/029291; WO 2003/055526; WO 2003/084477; WO 2003/094858; WO 2004/002417; WO 2004/002424; WO 2004/009627; WO 2004/024761; WO 2004/033651; WO 2004/035603; WO 2004/043382; WO 2004/101600; WO 2004/101606; WO 2004/101611; WO 2004/106373; WO 2004/018667; WO 2005/001025; WO 2005/001136; WO 2005/021579; WO 2005/025606; WO 2005/032460; WO 2005/051327; WO 2005/063808; WO 2005/063809; WO 2005/070451; WO 2005/081687; WO 2005/084711; WO 2005/103076; WO 2005/100403; WO 2005/092369; WO 2006/50959; WO 2006/02646; and WO 2006/29094.

Examples of other pharmaceutical products for use with the device may include, but are not limited to, antibodies such as Vectibix^{®} (panitumumab), Xgeva^{™} (denosumab) and Prolia^{™} (denosamab); other biological agents such as Enbrel^{®} (etanercept, TNF-receptor /Fc fusion protein, TNF blocker), Neulasta^{®} (pegfilgrastim, pegylated filgastrim, pegylated G-CSF, pegylated hu-Met-G-CSF), Neupogen^{®} (filgrastim , G-CSF, hu-MetG-CSF), and Nplate^{®} (romiplostim); small molecule drugs such as Sensipar^{®} (cinacalcet). The device may also be used with a therapeutic antibody, a polypeptide, a protein or other chemical, such as an iron, for example, ferumoxytol, iron dextrans, ferric glyconate, and iron sucrose. The pharmaceutical product may be in liquid form, or reconstituted from lyophilized form.

Among particular illustrative proteins are the specific proteins set forth below, including fusions, fragments, analogs, variants or derivatives thereof:
OPGL specific antibodies, peptibodies, and related proteins, and the like (also referred to as RANKL specific antibodies, peptibodies and the like), including fully humanized and human OPGL specific antibodies, particularly fully humanized monoclonal antibodies, including but not limited to the antibodies described in PCT Publication No. WO 03/002713, as to OPGL specific antibodies and antibody related proteins, particularly those having the sequences set forth therein, particularly, but not limited to, those denoted therein: 9H7; 18B2; 2D8; 2E11; 16E1; and 22B3, including the OPGL specific antibodies having either the light chain of SEQ ID NO:2 as set forth therein in Figure 2 and/or the heavy chain of SEQ ID NO:4, as set forth therein in Figure 4;
Myostatin binding proteins, peptibodies, and related proteins, and the like, including myostatin specific peptibodies, particularly those described in U.S. Publication No. 2004/0181033 and PCT Publication No. WO 2004/058988, particularly in parts pertinent to myostatin specific peptibodies, including but not limited to peptibodies of the mTN8-19 family, including those of SEQ ID NOS:305-351, including TN8-19-1 through TN8-19-40,
   TN8-19 con1 and TN8-19 con2; peptibodies of the mL2 family of SEQ ID NOS:357-383; the mL15 family of SEQ ID NOS:384-409; the mL17 family of SEQ ID NOS:410-438; the mL20 family of SEQ ID NOS:439-446; the mL21 family of SEQ ID NOS:447-452; the mL24 family of SEQ ID NOS:453-454; and those of SEQ ID NOS:615-631, as disclosed in the foregoing publication;
IL-4 receptor specific antibodies, peptibodies, and related proteins, and the like, particularly those that inhibit activities mediated by binding of IL-4 and/or IL-13 to the receptor, including those described in PCT Publication No. WO 2005/047331 or PCT Application No. PCT/US2004/37242 and in U.S. Publication No. 2005/112694, particularly in parts pertinent to IL-4 receptor specific antibodies, particularly such antibodies as are described therein, particularly, and without limitation, those designated therein: L1H1; L1H2; L1H3; L1H4; L1H5; L1H6; L1H7; L1H8; L1H9; L1H10; L1H11; L2H1; L2H2; L2H3; L2H4; L2H5; L2H6; L2H7; L2H8; L2H9; L2H10; L2H11; L2H12; L2H13; L2H14; L3H1; L4H1; L5H1; L6H1, as disclosed in the foregoing publication;
Interleukin 1-receptor 1 ("IL1-R1") specific antibodies, peptibodies, and related proteins, and the like, including but not limited to those described in U.S. Publication No. 2004/097712, in parts pertinent to IL1-R1 specific binding proteins, monoclonal antibodies in particular, especially, without limitation, those designated therein: 15CA, 26F5, 27F2, 24E12, and 10H7, as disclosed in the aforementioned publication;
Ang2 specific antibodies, peptibodies, and related proteins, and the like, including but not limited to those described in PCT Publication No. WO 03/057134 and U.S. Publication No. 2003/0229023, particularly in parts pertinent to Ang2 specific antibodies and peptibodies and the like, especially those of sequences described therein and including but not limited to: L1(N); L1(N) WT; L1(N) 1K WT; 2xL1(N); 2xL1(N) WT; Con4 (N), Con4 (N) 1K WT, 2xCon4 (N) 1K; L1C; L1C 1K; 2xL1C; Con4C; Con4C 1K; 2xCon4C 1K; Con4-L1 (N); Con4-L1C; TN-12-9 (N); C17 (N); TN8-8(N); TN8-14 (N); Con 1 (N), also including anti-Ang 2 antibodies and formulations such as those described in PCT Publication No. WO 2003/030833, particularly Ab526; Ab528; Ab531; Ab533; Ab535; Ab536; Ab537; Ab540; Ab543; Ab544; Ab545; Ab546; A551; Ab553; Ab555; Ab558; Ab559; Ab565; AbF1AbFD; AbFE; AbFJ; AbFK; AbG1D4; AbGC1E8; AbH1C12; AblA1; AbIF; AblK, AbIP; and AbIP, in their various permutations as described therein, as disclosed in the foregoing publication;
NGF specific antibodies, peptibodies, and related proteins, and the like including, in particular, but not limited to those described in U.S. Publication No. 2005/0074821 and U.S. Patent No. 6,919,426, particularly as to NGF-specific antibodies and related proteins in this regard, including in particular, but not limited to, the NGF-specific antibodies therein designated 4D4, 4G6, 6H9, 7H2, 14D10 and 14D11, as disclosed in the foregoing publication;
CD22 specific antibodies, peptibodies, and related proteins, and the like, such as those described in U.S. Patent No. 5,789,554, as to CD22 specific antibodies and related proteins, particularly human CD22 specific antibodies, such as but not limited to humanized and fully human antibodies, including but not limited to humanized and fully human monoclonal antibodies, particularly including but not limited to human CD22 specific IgG antibodies, such as, for instance, a dimer of a human-mouse monoclonal hLL2 gamma-chain disulfide linked to a human-mouse monoclonal hLL2 kappa-chain, including, but limited to, for example, the human CD22 specific fully humanized antibody in Epratuzumab, CAS registry number 501423-23-0;
IGF-1 receptor specific antibodies, peptibodies, and related proteins, and the like, such as those described in PCT Publication No. WO 06/069202, as to IGF-1 receptor specific antibodies and related proteins, including but not limited to the IGF-1 specific antibodies therein designated L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, L52H52, and IGF-1R-binding fragments and derivatives thereof, as disclosed in the foregoing publication;

Also among non-limiting examples of anti-IGF-1R antibodies for use in the methods and compositions of the present invention are each and all of those described in:
(i) U.S. Publication No. 2006/0040358 (published February 23, 2006), 2005/0008642 (published January 13, 2005), 2004/0228859 (published November 18, 2004), including but not limited to, for instance, antibody 1A (DSMZ Deposit No. DSM ACC 2586), antibody 8 (DSMZ Deposit No. DSM ACC 2589), antibody 23 (DSMZ Deposit No. DSM ACC 2588) and antibody 18 as described therein;
(ii) PCT Publication No. WO 06/138729 (published December 28, 2006) and WO 05/016970 (published February 24, 2005), and Lu et al. (2004), J. Biol. Chem. 279:2856-2865, including but not limited to antibodies 2F8, A12, and IMC-A12 as described therein;
(iii) PCT Publication No. WO 07/012614 (published February 1, 2007), WO 07/000328 (published January 4, 2007), WO 06/013472 (published February 9, 2006), WO 05/058967 (published June 30, 2005), and WO 03/059951 (published July 24, 2003);
(iv) U.S. Publication No. 2005/0084906 (published April 21, 2005), including but not limited to antibody 7C10, chimaeric antibody C7C10, antibody h7C10, antibody 7H2M, chimaeric antibody *7C10, antibody GM 607, humanized antibody 7C10 version 1, humanized antibody 7C10 version 2, humanized antibody 7C10 version 3, and antibody 7H2HM, as described therein;
(v) U.S. Publication Nos. 2005/0249728 (published November 10, 2005), 2005/0186203 (published August 25, 2005), 2004/0265307 (published December 30, 2004), and 2003/0235582 (published December 25, 2003) and Maloney et al. (2003), Cancer Res. 63:5073-5083, including but not limited to antibody EM164, resurfaced EM164, humanized EM164, huEM164 v1.0, huEM164 v1.1, huEM164 v1.2, and huEM164 v1.3 as described therein;
(vi) U.S. Patent No. 7,037,498 (issued May 2, 2006), U.S. Publication Nos. 2005/0244408 (published November 30, 2005) and 2004/0086503 (published May 6, 2004), and Cohen, et al. (2005), Clinical Cancer Res. 11:2063-2073, e.g., antibody CP-751,871, including but not limited to each of the antibodies produced by the hybridomas having the ATCC accession numbers PTA-2792, PTA-2788, PTA-2790, PTA-2791, PTA-2789, PTA-2793, and antibodies 2.12.1, 2.13.2, 2.14.3, 3.1.1, 4.9.2, and 4.17.3, as described therein;
(vii) U.S. Publication Nos. 2005/0136063 (published June 23, 2005) and 2004/0018191 (published January 29, 2004), including but not limited to antibody 19D12 and an antibody comprising a heavy chain encoded by a polynucleotide in plasmid 15H12/19D12 HCA (y4), deposited at the ATCC under number PTA-5214, and a light chain encoded by a polynucleotide in plasmid 15H12/19D12 LCF (κ), deposited at the ATCC under number PTA-5220, as described therein; and
(viii) U.S. Publication No. 2004/0202655 (published October 14, 2004), including but not limited to antibodies PINT-6A1, PINT-7A2, PINT-7A4, PINT-7A5, PINT-7A6, PINT-8A1, PINT-9A2, PINT-11A1, PINT-11A2, PINT-11A3, PINT-11A4, PINT-11A5, PINT-11A7, PINT-11A12, PINT-12A1, PINT-12A2, PINT-12A3, PINT-12A4, and PINT-12A5, as described therein; particularly as to the aforementioned antibodies, peptibodies, and related proteins and the like that target IGF-1 receptors;

B-7 related protein 1 specific antibodies, peptibodies, related proteins and the like ("B7RP-1," also is referred to in the literature as B7H2, ICOSL, B7h, and CD275), particularly B7RP-specific fully human monoclonal IgG2 antibodies, particularly fully human IgG2 monoclonal antibody that binds an epitope in the first immunoglobulin-like domain of B7RP-1, especially those that inhibit the interaction of B7RP-1 with its natural receptor, ICOS, on activated T cells in particular, especially, in all of the foregoing regards, those disclosed in U.S. Publication No. 2008/0166352 and PCT Publication No. WO 07/011941, as to such antibodies and related proteins, including but not limited to antibodies designated therein as follow: 16H (having light chain variable and heavy chain variable sequences SEQ ID NO:1 and SEQ ID NO:7 respectively therein); 5D (having light chain variable and heavy chain variable sequences SEQ ID NO:2 and SEQ ID NO:9 respectively therein); 2H (having light chain variable and heavy chain variable sequences SEQ ID NO:3 and SEQ ID NO:10 respectively therein); 43H (having light chain variable and heavy chain variable sequences SEQ ID NO:6 and SEQ ID NO:14 respectively therein); 41H (having light chain variable and heavy chain variable sequences SEQ ID NO:5 and SEQ ID NO:13 respectively therein); and 15H (having light chain variable and heavy chain variable sequences SEQ ID NO:4 and SEQ ID NO:12 respectively therein), as disclosed in the foregoing publication;
IL-15 specific antibodies, peptibodies, and related proteins, and the like, such as, in particular, humanized monoclonal antibodies, particularly antibodies such as those disclosed in U.S. Publication Nos. 2003/0138421; 2003/023586; and 2004/0071702; and U.S. Patent No. 7,153,507, as to IL-15 specific antibodies and related proteins, including peptibodies, including particularly, for instance, but not limited to, HuMax IL-15 antibodies and related proteins, such as, for instance, 146B7;
IFN gamma specific antibodies, peptibodies, and related proteins and the like, especially human IFN gamma specific antibodies, particularly fully human anti-IFN gamma antibodies, such as, for instance, those described in U.S. Publication No. 2005/0004353, particularly, for example, the antibodies therein designated 1118; 1118*; 1119; 1121; and 1121*. The entire sequences of the heavy and light chains of each of these antibodies, as well as the sequences of their heavy and light chain variable regions and complementarity determining regions, are disclosed in the foregoing publication and in Thakur et al. (1999), Mol. Immunol. 36:1107-1115. In addition, description of the properties of these antibodies is provided in the foregoing publication. Specific antibodies include those having the heavy chain of SEQ ID NO:17 and the light chain of SEQ ID NO:18; those having the heavy chain variable region of SEQ ID NO:6 and the light chain variable region of SEQ ID NO:8; those having the heavy chain of SEQ ID NO:19 and the light chain of SEQ ID NO:20; those having the heavy chain variable region of SEQ ID NO:10 and the light chain variable region of SEQ ID NO:12; those having the heavy chain of SEQ ID NO:32 and the light chain of SEQ ID NO:20; those having the heavy chain variable region of SEQ ID NO:30 and the light chain variable region of SEQ ID NO:12; those having the heavy chain sequence of SEQ ID NO:21 and the light chain sequence of SEQ ID NO:22; those having the heavy chain variable region of SEQ ID NO:14 and the light chain variable region of SEQ ID NO:16; those having the heavy chain of SEQ ID NO:21 and the light chain of SEQ ID NO:33; and those having the heavy chain variable region of SEQ ID NO:14 and the light chain variable region of SEQ ID NO:31, as disclosed in the foregoing publication. A specific antibody contemplated is antibody 1119 as disclosed in the foregoing U.S. publication and having a complete heavy chain of SEQ ID NO:17 as disclosed therein and having a complete light chain of SEQ ID NO:18 as disclosed therein;
TALL-1 specific antibodies, peptibodies, and the related proteins, and the like, and other TALL specific binding proteins, such as those described in U.S. Publication Nos. 2003/0195156 and 2006/0135431, as to TALL-1 binding proteins, particularly the molecules of Tables 4 and 5B, as disclosed in the foregoing publications;
Parathyroid hormone ("PTH") specific antibodies, peptibodies, and related proteins, and the like, such as those described in U.S. Patent No. 6,756,480, particularly in parts pertinent to proteins that bind PTH;
Thrombopoietin receptor ("TPO-R") specific antibodies, peptibodies, and related proteins, and the like, such as those described in U.S. Patent No. 6,835,809, particularly in parts pertinent to proteins that bind TPO-R;
Hepatocyte growth factor ("HGF") specific antibodies, peptibodies, and related proteins, and the like, including those that target the HGF/SF:cMet axis (HGF/SF:c-Met), such as the fully human monoclonal antibodies that neutralize hepatocyte growth factor/scatter (HGF/SF) described in U.S. Publication No. 2005/0118643 and PCT Publication No. WO 2005/017107, huL2G7 described in U.S. Patent No. 7,220,410 and OA-5d5 described in U.S. Patent Nos. 5,686,292 and 6,468,529 and in PCT Publication No. WO 96/38557, particularly in parts pertinent to proteins that bind HGF;
TRAIL-R2 specific antibodies, peptibodies, related proteins and the like, such as those described in U.S. Patent No. 7,521,048, particularly in parts pertinent to proteins that bind TRAIL-R2;
Activin A specific antibodies, peptibodies, related proteins, and the like, including but not limited to those described in U.S. Publication No. 2009/0234106, particularly in parts pertinent to proteins that bind Activin A;
TGF-beta specific antibodies, peptibodies, related proteins, and the like, including but not limited to those described in U.S. Patent No. 6,803,453 and U.S. Publication No. 2007/0110747, particularly in parts pertinent to proteins that bind TGF-beta;
Amyloid-beta protein specific antibodies, peptibodies, related proteins, and the like, including but not limited to those described in PCT Publication No. WO 2006/081171, particularly in parts pertinent to proteins that bind amyloid-beta proteins. One antibody contemplated is an antibody having a heavy chain variable region comprising SEQ ID NO:8 and a light chain variable region having SEQ ID NO:6 as disclosed in the foregoing publication;
c-Kit specific antibodies, peptibodies, related proteins, and the like, including but not limited to those described in U.S. Publication No. 2007/0253951, particularly in parts pertinent to proteins that bind c-Kit and/or other stem cell factor receptors;
OX40L specific antibodies, peptibodies, related proteins, and the like, including but not limited to those described in U.S. Publication No. 2006/0002929, particularly in parts pertinent to proteins that bind OX40L and/or other ligands of the OX40 receptor; and
Other exemplary proteins, including Activase^{®} (alteplase, tPA); Aranesp^{®} (darbepoetin alfa); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti- α4β7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Genotropin^{®} (somatropin, Human Growth Hormone); Herceptin^{®} (trastuzumab, anti-HER2/neu (erbB2) receptor mAb); Humatrope^{®} (somatropin, Human Growth Hormone); Humira^{®} (adalimumab); insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Neulasta^{®} (pegylated filgastrim, pegylated G-CSF, pegylated hu-Met-G-CSF); Neupogen^{®} (filgrastim , G-CSF, hu-MetG-CSF); Orthoclone OKT3^{®} (muromonab-CD3, anti-CD3 monoclonal antibody); Procrit^{®} (epoetin alfa); Remicade^{®} (infliximab, anti-TNFα monoclonal antibody); Reopro^{®} (abciximab, anti-GP IIb/IIia receptor monoclonal antibody); Actemra^{®} (anti-IL6 Receptor mAb); Avastin^{®} (bevacizumab), HuMax-CD4 (zanolimumab); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 146B7-CHO (anti-IL15 antibody, see U.S. Patent No. 7,153,507); Tysabri^{®} (natalizumab, anti-α4integrin mAb); Valortim^{®} (MDX-1303, anti-B. anthracis protective antigen mAb); ABthrax^{™}; Vectibix^{®} (panitumumab); Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Rα mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-1103); anti-IGF1R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); anti-LLY antibody; BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRα antibody (IMC-3G3); anti-TGFβ mAb (GC-1008); anti-TRAIL Receptor-2 human mAb (HGS-ETR2); anti-TWEAK mAb; anti-VEGFR/Flt-1 mAb; anti-ZP3 mAb (HuMax-ZP3); NVS Antibody #1; and NVS Antibody #2.

Also included can be a sclerostin antibody, such as but not limited to romosozumab, blosozumab, or BPS 804 (Novartis). Further included can be therapeutics such as rilotumumab, bixalomer, trebananib, ganitumab, conatumumab, motesanib diphosphate, brodalumab, vidupiprant, panitumumab, denosumab, NPLATE, PROLIA, VECTIBIX or XGEVA. Additionally, included in the device can be a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab), as well as molecules, variants, analogs or derivatives thereof as disclosed in the following patents or patent applications: U.S. Patent No. 8,030,547, U.S. Publication No. 2013/0064825, WO2008/057457, WO2008/057458, WO2008/057459, WO2008/063382, WO2008/133647, WO2009/100297, WO2009/100318, WO2011/037791, WO2011/053759, WO2011/053783, WO2008/125623, WO2011/072263, WO2009/055783, WO2012/0544438, WO2010/029513, WO2011/111007, WO2010/077854, WO2012/088313, WO2012/101251, WO2012/101252, WO2012/101253, WO2012/109530, and WO2001/031007.

Also included can be talimogene laherparepvec or another oncolytic HSV for the treatment of melanoma or other cancers. Examples of oncolytic HSV include, but are not limited to talimogene laherparepvec (U.S. Patent Nos. 7,223,593 and 7,537,924); OncoVEXGALV/CD (U.S. Pat. No. 7,981,669); OrienX010 (Lei et al. (2013), World J. Gastroenterol., 19:5138-5143); G207, 1716; NV1020; NV12023; NV1034 and NV1042 (Vargehes et al. (2002), Cancer Gene Ther., 9(12):967-978).

Also included are TIMPs. TIMPs are endogenous tissue inhibitors of metalloproteinases (TIMPs) and are important in many natural processes. TIMP-3 is expressed by various cells or and is present in the extracellular matrix; it inhibits all the major cartilage-degrading metalloproteases, and may play a role in role in many degradative diseases of connective tissue, including rheumatoid arthritis and osteoarthritis, as well as in cancer and cardiovascular conditions. The amino acid sequence of TIMP-3, and the nucleic acid sequence of a DNA that encodes TIMP-3, are disclosed in U.S. Patent No. 6,562,596, issued May 13, 2003. Description of TIMP mutations can be found in U.S. Publication No. 2014/0274874 and PCT Publication No. WO 2014/152012.

Also included are antagonistic antibodies for human calcitonin gene-related peptide (CGRP) receptor and bispecific antibody molecule that target the CGRP receptor and other headache targets. Further information concerning these molecules can be found in PCT Application No. WO 2010/075238.

Additionally, bispecific T cell engager (BiTE^{®}) antibodies, e.g. BLINCYTO^{®} (blinatumomab), can be used in the device. Alternatively, included can be an APJ large molecule agonist e.g., apelin or analogues thereof in the device. Information relating to such molecules can be found in PCT Publication No. WO 2014/099984.

In certain embodiments, the medicament comprises a therapeutically effective amount of an anti-thymic stromal lymphopoietin (TSLP) or TSLP receptor antibody. Examples of anti-TSLP antibodies that may be used in such embodiments include, but are not limited to, those described in U.S. Patent Nos. 7,982,016, and 8,232,372, and U.S. Publication No. 2009/0186022. Examples of anti-TSLP receptor antibodies include, but are not limited to, those described in U.S. Patent No. 8,101,182. In particularly preferred embodiments, the medicament comprises a therapeutically effective amount of the anti-TSLP antibody designated as A5 within U.S. Patent No. 7,982,016.

The detailed description is to be construed as exemplary only and does not describe every possible embodiment of the invention because describing every possible embodiment would be impractical, if not impossible. Numerous alternative embodiments could be implemented, using either current technology or technology developed after the filing date of this patent that would still fall within the scope of the claims defining the invention.

## Claims

1. A wearable drug delivery device (100, 200, 300, 400, 500, 600, 700, 800, 900, 1000, 1100, 1200), comprising:
an injector including a housing (106, 206, 306, 406, 506, 606, 706, 806, 906, 1006, 1106, 1206), a reservoir, a needle or cannula, and a drive mechanism for urging drug product out of the reservoir, through the needle or cannula and to a patient, the reservoir, the needle or cannula, and the drive mechanism being at least partially disposed in the housing; and
an adhesive applicator (104, 204, 304, 404, 504, 604, 704, 804, 904, 1004, 1104, 1204) including a non-woven material fixed to the bottom surface of the injector having a dimension that is larger than the injector itself such that the adhesive applicator includes a perimeter portion that encircles a perimeter of the injector,
the housing including a sidewall with at least one side gripping feature (108, 208, 308, 408, 508, 608, 708, 808, 908, 1008, 1108, 1208); and
wherein the adhesive applicator includes a radial slit (112, 613) in the perimeter portion for facilitating deformation to conform to the contours of a patient's skin.

2. The device of claim 1, wherein the adhesive applicator further includes at least one tab that extends beyond the perimeter of the injector.

3. The device of claim 1 or 2, wherein the side gripping feature includes one or more of the following:
a rib;
a plurality of ribs;
a recess;
a plurality of recesses;
a recess that extends to a bottom surface of the injector.

4. The device of any one of claims 1-3, further comprising a pull tab (310) extending from the perimeter portion of the adhesive applicator.

5. The device of claim 4, wherein the pull tab (310) includes an adhesive with less tack than the remainder of the adhesive applicator.

6. The device of any one of claims 4-5, wherein the pull tab (310) includes a color or shading that is distinct from a color or shading of the remainder of the adhesive applicator.

7. The wearable drug delivery device of any one of claims 1-6, wherein the adhesive applicator has an X shaped profile with four pull tabs (210) extending beyond a perimeter of the injector.

8. The wearable drug delivery device of any one of claims 1-7, wherein,:
the perimeter portion completely encircles a perimeter of the injector; and
the wearable drug delivery device includes a pull ring (408) attached to the injector or the adhesive applicator, the pull ring including a first position adhered to a top surface of the injector and a second position released from the top surface of the injector for facilitating removal of the injector from a patient after drug delivery has occurred.

9. The wearable drug delivery device of any one of claims 1-8, wherein:
the perimeter portion completely encircles a perimeter of the injector, and
the adhesive applicator including a skeletal structure (506a) interconnecting a plurality of primary bonding structures, the skeletal structure having a stiffness greater than a stiffness of the primary bonding structures.

10. The wearable drug delivery device of any one of claims 1-9, wherein:
the adhesive applicator is removably attached to the injector; and
the wearable drug delivery device includes a pull chord (408,808) removably connecting the injector to the adhesive applicator, the pull chord being removable to separate the injector from the adhesive applicator after drug delivery has occurred.

11. The wearable drug delivery device of any one of claims 1-10, further comprising a pull ring (412, 812, 912) removably connecting the injector to the adhesive applicator, the pull ring being removable to separate the injector from the adhesive applicator after drug delivery has occurred.

12. The wearable drug delivery device of any one of claims 1-11, wherein:
the adhesive applicator is removably attached to the injector, and
the adhesive applicator includes a perforated seam (916) that extends around at least a portion of the injector for facilitating removal of the injector from the patient after drug delivery has occurred.

13. The wearable drug delivery device of any one of claims 1-12, wherein:
the adhesive applicator is removably attached to the injector; and
the wearable drug delivery device includes a pair of edge tabs (1012) extending up from the adhesive applicator and being removably adhered to a top surface of the injector for securing the injector to the adhesive applicator, the edge tabs being removable from the top surface of the applicator after drug delivery has occurred to allow the injector to be separated from the adhesive applicator.

## Patentansprüche

1. Tragbare Arzneimittelabgabevorrichtung (100, 200, 300, 400, 500, 600, 700, 800, 900, 1000, 1100, 1200), die umfasst:
einen Injektor, der ein Gehäuse (106, 206, 306, 406, 506, 606, 706, 806, 906, 1006, 1106, 1206), ein Reservoir, eine Nadel oder Kanüle und einen Antriebsmechanismus zum Drängen des Arzneimittels aus dem Reservoir durch die Nadel oder Kanüle hindurch und zu einem Patienten beinhaltet, wobei das Reservoir, die Nadel oder Kanüle und der Antriebsmechanismus wenigstens teilweise in dem Gehäuse angeordnet sind; und
einen Klebstoffapplikator (104, 204, 304, 404, 504, 604, 704, 804, 904, 1004, 1104, 1204), der einen Vliesstoff beinhaltet, der an der Unterseite des Injektors befestigt ist und eine Abmessung aufweist, die größer ist als der Injektor selbst, sodass der Klebstoffapplikator einen Umfangsabschnitt beinhaltet, der den Umfang des Injektors umgibt, wobei
das Gehäuse eine Seitenwand mit wenigstens einem seitlichen Greifmerkmal (108, 208, 308, 408, 508, 608, 708, 808, 908, 1008, 1108, 1208) beinhaltet; und
wobei der Klebstoffapplikator einen radialen Schlitz (112, 613) in dem Umfangsabschnitt beinhaltet, um die Verformung zu erleichtern, um sich an die Konturen der Haut eines Patienten anzupassen.

2. Vorrichtung nach Anspruch 1, wobei der Klebstoffapplikator ferner wenigstens eine Lasche beinhaltet, die sich über den Umfang des Injektors hinaus erstreckt.

3. Vorrichtung nach Anspruch 1 oder 2, wobei das seitliche Greifmerkmal eines oder mehrere der folgenden Merkmale beinhaltet:
eine Rippe;
eine Mehrzahl von Rippen;
eine Aussparung;
eine Mehrzahl von Aussparungen;
eine Aussparung, die sich bis zu der Unterseite des Injektors erstreckt.

4. Vorrichtung nach einem der Ansprüche 1-3, die ferner eine Zuglasche (310) beinhaltet, die sich von dem Umfangsabschnitt des Klebstoffapplikators erstreckt.

5. Vorrichtung nach Anspruch 4, wobei die Zuglasche (310) einen Klebstoff mit geringerer Klebkraft als der Rest des Klebstoffapplikators beinhaltet.

6. Vorrichtung nach einem der Ansprüche 4-5, wobei die Zuglasche (310) eine Farbe oder Schattierung beinhaltet, die sich von der Farbe oder Schattierung des Rests des Klebstoffapplikators unterscheidet.

7. Tragbare Arzneimittelabgabevorrichtung nach einem der Ansprüche 1-6, wobei der Klebstoffapplikator ein X-förmiges Profil mit vier Zuglaschen (210) aufweist, die sich über den Umfang des Injektors hinaus erstrecken.

8. Tragbare Arzneimittelabgabevorrichtung nach einem der Ansprüche 1-7, wobei:
der Umfangsabschnitt den Umfang des Injektors vollständig umgibt; und
die tragbare Arzneimittelabgabevorrichtung einen Zugring (408) beinhaltet, der an dem Injektor oder an dem Klebstoffapplikator angebracht ist, wobei der Zugring eine erste Position an der Oberseite des Injektors anhaftend und eine zweite Position von der Oberseite des Injektors freigegeben beinhaltet, um die Entfernung des Injektors von einem Patienten zu erleichtern, nachdem eine Arzneimittelabgabe aufgetreten ist.

9. Tragbare Arzneimittelabgabevorrichtung nach einem der Ansprüche 1-8, wobei:
der Umfangsabschnitt den Umfang des Injektors vollständig umgibt, und
der Klebstoffapplikator eine Skelettstruktur (506a) beinhaltet, die eine Mehrzahl von primären Verbindungsstrukturen miteinander verbindet, wobei die Skelettstruktur eine größere Steifigkeit als eine Steifigkeit der primären Verbindungsstrukturen aufweist.

10. Tragbare Arzneimittelabgabevorrichtung nach einem der Ansprüche 1-9, wobei:
der Klebstoffapplikator entfernbar an dem Injektor befestigt ist; und
die tragbare Arzneimittelabgabevorrichtung eine Zugschnur (408, 808) beinhaltet, die den Injektor entfernbar mit dem Klebstoffapplikator verbindet, wobei die Zugschnur entfernbar ist, um den Injektor von dem Klebstoffapplikator zu trennen, nachdem eine Arzneimittelabgabe aufgetreten ist.

11. Tragbare Arzneimittelabgabevorrichtung nach einem der Ansprüche 1-10, die ferner einen Zugring (412, 812, 912) umfasst, der den Injektor entfernbar mit dem Klebstoffapplikator verbindet, wobei der Zugring entfernbar ist, um den Injektor von dem Klebstoffapplikator zu trennen, nachdem eine Arzneimittelabgabe aufgetreten ist.

12. Tragbare Arzneimittelabgabevorrichtung nach einem der Ansprüche 1-11, wobei:
der Klebstoffapplikator entfernbar an dem Injektor angebracht ist, und
der Klebstoffapplikator eine perforierte Naht (916) beinhaltet, die sich um wenigstens einen Abschnitt des Injektors erstreckt, um das Entfernen des Injektors von dem Patienten zu erleichtern, nachdem eine Arzneimittelabgabe aufgetreten ist.

13. Tragbare Arzneimittelabgabevorrichtung nach einem der Ansprüche 1-12, wobei:
der Klebstoffapplikator entfernbar an dem Injektor angebracht ist; und
die tragbare Arzneimittelabgabevorrichtung ein Paar Randlaschen (1012) beinhaltet, die sich von dem Klebstoffapplikator nach oben erstrecken und entfernbar an eine Oberseite des Injektors geklebt sind, um den Injektor am Klebstoffapplikator zu sichern, wobei die Randlaschen von der Oberseite des Applikators entfernbar sind, nachdem eine Arzneimittelabgabe aufgetreten ist, um zu ermöglichen, den Injektor von dem Klebstoffapplikator zu trennen.

## Revendications

1. Dispositif portable d'administration de médicament (100, 200, 300, 400, 500, 600, 700, 800, 900, 1000, 1100, 1200), comprenant ;
un injecteur comportant un boîtier (106, 206, 306, 406, 506, 606, 706, 806, 906, 1006, 1106, 1206), un réservoir, une aiguille ou une canule, et un mécanisme d'entraînement pour pousser un produit médicamenteux hors du réservoir, à travers l'aiguille ou la canule et jusqu'à un patient, le réservoir, l'aiguille ou la canule, et le mécanisme d'entraînement étant au moins partiellement disposés dans le boîtier ; et
un applicateur adhésif (104, 204, 304, 404, 504, 604, 704, 804, 904, 1004, 1104, 1204) comportant un matériau non tissé fixé à la surface inférieure de l'injecteur ayant une dimension qui est plus grande que l'injecteur lui-même de sorte que l'applicateur adhésif comporte une partie périmétrique qui encercle un périmètre de l'injecteur,
le boîtier comportant une paroi latérale avec au moins un accessoire de préhension latéral (108, 208, 308, 408, 508, 608, 708, 808, 908, 1008, 1108, 1208) ; et
dans lequel l'applicateur adhésif comporte une fente radiale (112, 613) dans la partie périmétrique pour faciliter la déformation pour s'adapter aux contours de la peau d'un patient.

2. Dispositif selon la revendication 1, dans lequel l'applicateur adhésif comporte en outre au moins une languette qui s'étend au-delà du périmètre de l'injecteur.

3. Dispositif selon la revendication 1 ou 2, dans lequel l'accessoire de préhension latéral comporte un ou plusieurs des éléments suivants :
une nervure ;
une pluralité de nervures ;
un évidement ;
une pluralité d'évidements ;
un évidement qui s'étend jusqu'à une surface inférieure de l'injecteur.

4. Dispositif selon l'une quelconque des revendications 1 à 3, comprenant en outre une languette à tirer (310) s'étendant à partir de la partie périmétrique de l'applicateur adhésif.

5. Dispositif selon la revendication 4, dans lequel la languette à tirer (310) comporte un adhésif avec moins d'adhérence que le reste de l'applicateur adhésif.

6. Dispositif selon l'une quelconque des revendications 4 et 5, dans lequel la languette à tirer (310) comporte une couleur ou une nuance qui est différente de la couleur ou de la nuance du reste de l'applicateur adhésif.

7. Dispositif portable d'administration de médicament selon l'une quelconque des revendications 1 à 6, dans lequel l'applicateur adhésif présente un profil en forme de X avec quatre languettes à tirer (210) s'étendant au-delà d'un périmètre de l'injecteur.

8. Dispositif portable d'administration de médicament selon l'une quelconque des revendications 1 à 7, dans lequel :
la partie périmétrique encercle complètement un périmètre de l'injecteur ; et
le dispositif portable d'administration de médicament comporte un anneau à tirer (408) attaché à l'injecteur ou à l'applicateur adhésif, l'anneau à tirer comportant une première position adhérant à une surface supérieure de l'injecteur et une deuxième position libérée de la surface supérieure de l'injecteur pour faciliter le retrait de l'injecteur d'un patient après l'administration de médicament.

9. Dispositif portable d'administration de médicament selon l'une quelconque des revendications 1 à 8, dans lequel :
la partie périmétrique encercle complètement un périmètre de l'injecteur, et
l'applicateur adhésif comporte une structure squelettique (506a) reliant entre elles une pluralité de structures de liaison primaires, la structure squelettique ayant une rigidité supérieure à la rigidité des structures de liaison primaires.

10. Dispositif portable d'administration de médicament selon l'une quelconque des revendications 1 à 9, dans lequel :
l'applicateur adhésif est attaché amovible à l'injecteur ; et
le dispositif portable d'administration de médicament comprend un cordon à tirer amovible (408, 808) reliant de manière amovible l'injecteur à l'applicateur adhésif, le cordon à tirer étant amovible pour séparer l'injecteur de l'applicateur adhésif après l'administration de médicament.

11. Dispositif portable d'administration de médicament selon l'une quelconque des revendications 1 à 10, comprenant en outre un anneau à tirer (412, 812, 912) reliant de manière amovible l'injecteur à l'applicateur adhésif, l'anneau à tirer étant amovible pour séparer l'injecteur de l'applicateur adhésif après l'administration de médicament.

12. Dispositif portable d'administration de médicament selon l'une quelconque des revendications 1 à 11, dans lequel :
l'applicateur adhésif est attaché amovible à l'injecteur, et
l'applicateur adhésif comporte une couture perforée (916) qui s'étend autour d'au moins une partie de l'injecteur pour faciliter le retrait de l'injecteur du patient après l'administration de médicament.

13. Dispositif portable d'administration de médicament selon l'une quelconque des revendications 1 à 12, dans lequel :
l'applicateur adhésif est attaché amovible à l'injecteur ; et
le dispositif portable d'administration de médicament comporte une paire de languettes de bord (1012) s'étendant vers le haut à partir de l'applicateur adhésif et adhérant de manière amovible à une surface supérieure de l'injecteur pour arrimer l'injecteur à l'applicateur adhésif, les languettes de bord étant amovibles de la surface supérieure de l'applicateur après l'administration de médicament pour permettre à l'injecteur d'être séparé de l'applicateur adhésif.
